# EUROPEAN PATENT SPECIFICATION

(11) **EP 1 346 745 B1**
(45) Date of publication and mention of the grant of the patent: **19.07.2006**
(21) Application number: 03251700.5
(22) Date of filing: 19.03.2003
(51) Int. Cl.: A61M 25/00

(54) **Apparatus for treatment of tumor in the central nervous system (CNS)**
Vorrichtung für Behandlung eines Tumors im zentralen Nervensystem
Appareil pour traitement de tumeur dans le système nerveux central

(30) Priority: 21.03.2002 US 365828
(43) Date of publication of application: 24.09.2003
(73) Proprietor: ED. GEISTLICH SÖHNE AG FÜR CHEMISCHE INDUSTRIE, 6110 Wolhusen (CH)
(72) Inventor: Pfirrmann, Rolf W., 6006 Lucerne (CH); Stendel, Ruediger, Berlin 14163 (DE)
(74) Representative: Marsden, John Christopher

(56) References cited:
- WO-A-01/39762
- WO-A-02/07810
- US-A- 6 030 358
- US-A- 6 093 180

## Description

### Field of the Invention

The present invention relates to apparatus for treating tumors and/or infections of the central nervous system (CNS) and/or other parenchymal organs.

### Description of the Background

Taurolidine (Bis-(1,1-dioxoperhydro-1,2,4-thiadiazinyl-4)methane) was developed by Geistlich Pharma. It is a white crystalline substance, water soluble up to 2%. It is made up of two molecules of taurinamid and three molecules formaldehyde forming a two-ringed structure bridged by a methylene group.

Taurolidine has primarily an antibiotic and anti-endotoxin effect. It acts by a chemical reaction, so no microorganism resistance has been observed as yet. This effect of taurolidine is mediated by its active metabolites, which are donators of active methylol-groups. Methylol-Taurultam and Methylol-Taurinamide. The active methylol groups inactivate by reacting with the cell wall of bacteria and with the primary amino groups of endotoxins.

Additional effects of taurolidine were reported in the past inhibition of TNF and IL-1Beta in mononudear cells (Bedrosian 1991), inhibition of Tumor Necrosis Factor Toxicity, and inhibition of Peritoneal Tumor Cell Growth in Laparoscopic Surgery (Jacobi 1997).

Taurolidine solutions have been used as instillation or rinsing solutions of the abdominal cavity in cases of peritonitis. In post-operative instillations, conscious patients have reported as a side-effect irritation of the nerves of the peritoneum, and sometimes strong burning sensations which require intravenous administration of pain killers or anaesthesia. Monson et al. PCT International Publication Number WO 92/00743 discloses a selective direct inhibiting effect of Taurolidine and/or Taurultam on certain body tumors. (Monson JRT, Ramsey PS, Donohue JH. Preliminary evidence that taurolidine is anti-neoplasbc as well as anti-endotoxin and anti-microbial. Abstract Br J Surg 77(6) 1990, A711) on B16 melanoma cells and Meth A sarcoma cells in a mice model in vivo, and on fibroblastic tumor cells, LS174T (colon-) carcinoma cells and Jurkat (leukemic-) cells in vitro (international Patent PCT No. PCT/EP91/01269, international Publication Number WO 92/00743 PCT "Use of Taurolidine and/or Taurultam for the treatment of tumors"). However, primary tumors of the brain and medulla of the Central Nervous System (CNS) are very different from those of the body. Nerve cells differ significantly from cells of other organs, and have a much more complex construction. Nerve cells are characterized by a great number of branches which serve to transmit impulses and sensations, including dendrites for reception of impulses, and neurites or axons for emission of impulses. Neurogliae are glia-cells which are present in greater numbers than neurons, and render stability to the nerve cells. Glia-cells are responsible for metabolism and protection of sensitive nerve cells. The cells from which CNS tumors arise have a different metabolism as compared to other tumor cells. Metastases of CNS tumors outside the nervous system are very rare. Effective surgical treatment is often impossible since the tumors are located in functionally important areas, or spread diffusely.

Primary tumors of the brain and spinal cord arise from the different cell types of the CNS. These cell types are neurons, which are responsible for the neuronal function and the glial cells, which have supporting and nutritioning functions. According to the different subtypes of glial and neuronal cells, there are different types of CNS-tumors. The most common brain tumors arise from the glial cells. Various sub-types (astrozytoma, oligodendroglioma, ependymoma, etc.) are encompassed by the term "glioma".

Gliomas are the most common primary brain tumors. The incidence of gliomas is about 5/100,000 persons per year. More than 50% are glioblastoma, the most malignant form, which is responsible for more than 2.5% of the total tumor associated mortality. More than 95% of the patients die within 2 years following diagnosis despite aggressive therapy including surgery, radiotherapy and chemotherapy.

Brain tumors have some special characteristics as compared to "peripheral" tumors. They act as space occupying lesions, caused by the bony skull. This situation causes herniation and death when the tumor grows larger than can be accommodated. Furthermore, primary brain tumors often metastasize via the cerebrospinal fluid within the whole central nervous system. The brain tumor cells have a lower cohesion within the cell formation as compared to "peripheral" tumor cells (Jänisch W.: Pathologic der Geschwülste des Zentralnervensystems In: Klinische Neuropathologie, J. Cervós-Navarro and R. Ferszt (Eds.) Thieme, Stuttgart, New York, 1989). In addition, the metabolism of brain tumors are influenced by the blood/brain barrier.

Both types of tumors, glial and neuronal, can develop malignantly. Malignant gliomas are more frequent as compared to benign gliomas (85% vs. 15%). In the U.S. there are about 20,000 new glioma and medulloblastoma cases per year. The glioblastoma is most common (about 65% among astrocytoma).

Therapeutic options of primary CNS-tumors include surgery, radiotherapy and chemotherapy. Complete resection is often impossible because of poorly defined tumor borders and location within the brain area. Nearly all malignant glioma reoccur within months, 90% on the original site. Reoperation for a recurrent glioma typically extends survival by about 36 weeks (10 weeks with good quality of life). There is no well designed study regarding the beneficial effect of radiotherapy following glioma surgery. In patients older than 65 years, the median survival following tumor biopsy plus radiation is about 17 weeks, and following tumor removal plus radiation about 30 weeks (the peak incidence of glioblastoma is at an age of about 60 years). However, complete tumor removal plus radiatherapy is considered the reference standard in glioma therapy.

Chemotherapy using alkylating agents has a positive response rate of about 30%. A positive response generally extends the survival by 6-8 weeks. However, only about 50% of the patients treated with chemotherapy using alkylating agents are able to maintain regular activities.

Despite progress in diagnosis and treatment, the prognosis of patients with malignant primary CNS-tumors is still poor. The median survival of glioblastoma patients following optimal therapy including complete extirpation and radiation is less than about 10 months (about 1.6 years in grade III astrocytomas). The 1-year survival rate of patients with glioblastoma is about 35%, the 2-year survival rate about 8%.

Some primary malignant central nervous system tumors cannot be treated surgically because of their location or diffuse extension (gliomatosis, diffuse brain stem gliomas). Chemotherapy is not generally recommended, since the response rate on these alkylating agents (BCNU, CCNU, Procarbazine) is about 10% of patients (data from Greenberg MS. Handbook of Neurosurgery. Third edition 1994, Greenberg Graphics Inc., Lakeland, FL, USA). Heretofore, no therapy could be offered to those patients despite a palliative radiation. Thus, the therapy of primary malignant tumors of the central nervous system has been very unsatisfactory.

In addition, a variety of microdialysis methods and devices are known in other contexts. A number of illustrative methods and devices are shown in the following U.S. Patents: U.S. Patent No. 6,463,312 (entitled "Microdialysis-Probe Integrated with a Si-Chip); U.S. Patent No. 6,091,976 (entitled "Determination of Glucose Concentration In Tissue"); U.S. Patent No. 6,030,358 (entitled "Microcatheter and Method for Site Specific Therapy"); U.S. Patent No. 5,741,284 (entitled "Dialysis Combination and Microdialysis Probe and Insertion Device"); U.S. Patent No. 5,735,832 (entitled "Reinforced Microdialysis Probe"); U.S. Patent No. 5,706,806 (involving "an improved linear microdialysis probe assembly [having] a short semipermeable membrane portion containing a flexible, internal support or reinforcement fiber bonded to long lengths of inlet and outlet robing"); U.S. Patent No. 5,441,481 (involving "a microdialysis probe arranged to have a primary probe, e.g., an electrical probe, secured to it so that the microdialysis probe extends about and is concentric with the primary probe").

In addition to the foregoing patents, the following U.S. Patents of the present assignee are also referred to : U.S. Patent No. 6,488,912 (entitled "Treatment of Dentoalveolar Infections with Taurolidine And/Or Taurultam") ; and U.S. Patent No.6,258,797 (entilled "Combating Infection In Delivery Systems").

There remains a need in the art for new apparatus and methodologies for, among other things, treating tumors of the central nervous system and/or other parenchymal organs.

### SUMMARY OF THE INVENTION

The present invention relates to a microprobe apparatus as defined in the claims for the treatment and/or prophylaxis of tumors and/or infections in the central nervous system and/or other parenchymal organs in mammalian subjects. The apparatus may be used to provide an effective dose of taurolidine and/or taurultam for administration to a mammalian subject suffering from or at risk to growth of tumors of the central nervous system and/or other parenchymal organs.

Despite the irritation of the nerves of the peritoneum and strong burning sensations which have been side-effects of peritonitis post-operative instillations of Taurolidine, it surprisingly has been found that CNS nerve cells, including the particularly sensitive stem cells of embryo meningeal cells, remain unaffected following administration of Taurolidine/Taurultam solutions.

It was surprising to demonstrate a direct antineoplastic effect of Taurolidine and/or Taurultam on neuronal and glial tumor cell lines. This effect was very unexpected due to the quite different behavior of brain tumor cells as compared to other tumor cells, particularly concerning their response to chemotherapeutic agents. Furthermore, the antineoplastic effect of Taurolidine and/or Taurultam was thought only to be associated with the influence on cell adhesion molecules, which explains the prevention of metastatic tumor growth following endoscopic abdominal tumor surgery. A direct antineoplastic effect on brain tumor cells was very unexpected.

### BRIEF DESCRIPTION OF THE DRAWINGS

The following figures are shown by way of example and not limitation, in which:

FIG. 1 shows a microprobe formed with a semipermeable membrane according to some illustrative embodiments;

FIG. 2(A) shows a microprobe formed with a semipermeable membrane including a small bundle of tubes according to some illustrative embodiments;

FIG. 2(B) is a cross-sectional view of the microprobe shown In FIG. 2(A) taken along the line 2B-2B shown in FIG. 2(A); and

FIG. 3 shows a microprobe according to some illustrative embodiments implanted within a patient.

### DETAILED DESCRIPTION OF THE PREFERRED EMBODIMENTS

As described above, the present invention relates to microprobe apparatus for the treatment and/or prophylaxis of tumors and/or infections in the central nervous system and/or other parenchymal organs in mammalian subjects. The apparatus comprises a microdialysis device which is used to provide an effective dose of taurolidine and/or taurultam for administration to a mammalian subject suffering from or at risk to growth of tumors of the central nervous system and/or other parenchymal organs.

### The Preferred Solutions:

Taurolidine and Taurultam, its intermediate and active metabolite, are methylol transfer agents. They act by transferring methylol groups at the site of action. Both substances have low toxicity and are not cytotoxic against normal cells.

The terms Taurolidine and/or Taurultam as used herein are intended to refer to the compounds Taurolidine, Taurultam, Taurultam-glucose (as described below), and their substantial bioequivalents or agents which act in a substantially similar manner. For example, an aminoglycan derived from Taurultam and any other suitable derivate of Taurolidine and/or Taurultam can be utilized like Taurolidine and/or Taurultam according to the invention.

The term "treatment" as used herein is intended to refer to treatment, prophylaxis and/or suppression of CNS tumors and/or infections. The preferred embodiments are applicable to treatment of CNS tumors, which may include:
- Glioblastoma Multiforme (GBM)
- High grade gliomas
- Anaplastic oligodendroglioma
- Low grade gliomas
- Recurrent malignant gliomas
- Anaplastic astrocytoma
- Advanced metastatic melanoma
- Recurrent high grade primary brain tumors
- Primary central nervous system lymphoma
- Leptomeningeal dissemination of malignant glioma (meningeal gliomatosis).

Treatment takes place primarily in connection with surgical intervention, such as surgical removal of a CNS tumor, as well as postoperative local application of taurolidine and/or Taurultam solution while using the microprobe apparatus of the invention. Since the blood/brain barrier is passed by Taurolidine and/or Taurultam, it also may be appropriate to administer 2% taurolidine solutions or 3% Taurultam solutions intravenously through a central catheter. Here, in addition to the antineoplastic action, prevention of infection is also of great advantage for the patient. In this connection, dosage appropriately may be 15-20 g of taurolidine as a 2% solution through a central catheter daily for 7-8 days, or alternatively as 3% Taurultam solution, 20-30 g Taurultam daily, for 7-8 days with adults. This is intended to preserve or improve neurological function and health-related quality of life. For local application in connection with operations in the brain, glucose-based solutions, with or without electrolytes, and which additionally contain 0.2-1% Taurolidine,Taurultam or Taurultam-glucose, are preferred.

Basic treatment solutions preferably are modeled after cerebrospinal solution, contain glucose and electrolytes, are substantially isotonic to the extent possible and have a slightly alkaline pH value of about 7.3-7.35. The following ingredients may be included in a basic solution:
- Bicarbonate
- Sodium
- Potassium
- Calcium
- Magnesium
- Lactate
- Chloride
- Glucose
Taurolidine, Taurultam, Taurultam-glucose or the like are added to a basic solution.

### Exemplary Basic Solution:

A basic solution may, for example, be comprised of Cerebrospinal Fluid (CSF) components as shown in the following table.

| **CONSTITUENT** | **UNITS** | **CSF** | **PLASMA** | **CSF: plasma ratio** |
|---|---|---|---|---|
| osmolarity | mOsm/L | 295 | 295 | 1.0 |
| H₂O content | | 99% | 93% | |
| sodium | mEq/L | 138 | 138 | 1.0 |
| potassium | mEq/L | 2.8 | 4.5 | 0.6 |
| chloride | mEq/L | 119 | 102 | 1.2 |
| calcium | mEq/L | 2.1 | 4.8 | 0.4 |
| pCO₂ | mm HG | 47 | 41 | 1.1 |
| pH | | 7.33 | 7.41 | |
| pO₂ | mm Hg | 43 | 104 | 0.4 |
| glucose | mg/dl | 60 | 90 | 0.67 |
| lactate | mEq/L | 1.6 | 1.0 | 1.6 |

### Exemplary Amino-sugar/Taurultam-glucose Treatment Agent:

13.6 g Taurultam and 18 g of anhydrous glucose were weighed out into a 250 ml serum bottle, and 200 ml of distilled water were added. The solution obtained was heated to 100°C for 30 minutes. The clear solution was evaporated in a vacuum until dry. The residue was absorbed in 96% alcohol and placed in an Erlenmeyer flask overnight for forming crystals.

Amino-sugar/Taurultam-glucose crystallized out, and the crystals were suction filtered with a raw yield of 5.3 g.

From alcohol mixed with a few drops of water, white crystals were recrystaltized:
Melting point: 168° - 170° C.

| | | | | |
|---|---|---|---|---|
| Calculated: | C = 36.23 | H = 6.03 | N = 9.39 | S = 10.74% |
| Found: | C = 36.26 | H = 6.10 | N = 9.09 | S = 10.90% |

The IR spectrum corresponded NMR in DMSO₆ 200 MHZ. Sulfonamide NH coupling to its adjacent CH₂, one OH coupling to CH₂ and three OH's couplings to CH indicated internal loss of water and that the chain had cyclised to form a sugar.

### Illustrative solutions for use in the microprobe apparatus:

| **Solution 1** | 1000 ml contain: |
|---|---|
| Glucose monohydrate for injection purposes | 27.500 g |
| Sodium | 3.382 g |
| Potassium | 0.157 g |
| Ca⁺⁺ | 0.009 g |
| cr | 5.520 g |
| Taurultam | 0.5% |

The solution is slightly hypertonic.
The glucose can be replaced by 25 g levulose (fructose).
the solution is then insulin-independent.

| **Solution 2** | 1000 ml contain: |
|---|---|
| Sodium | 3.151 g |
| Potassium | 0.156 g |
| Ca⁺⁺ | 0.066 g |
| Mg⁺⁺ | 0.033 g |
| Cr | 3.900 g |
| Acetate | 2.173 g |
| Taurultam-glucose | 0.5% |

The pH value is set at pH 7.3.
The solutions 1 and 2 are filtered in an appropriately sterile manner with a 0.1 micron sterile filter and aseptically deposited in sterile infusion bottles.

| **Solution 3** | 1000 ml contain |
|---|---|
| Glucose monohydrate for injection purposes | 18.330 g |
| Sodium lactate | 2.460 g |
| Sodium chloride. | 2.800 g |
| Potassium chloride | 0.187 g |
| Calcium chloride 2 H₂O | 0.147 g |
| Magnesium chloride 6 H₂O | 0.152 g |
| Taurolidine | 1% |

The pH is set at 7.3. The solution is filtered in a sterile manner and aseptically deposited in 100 ml infusion bottles.

| **Solution 4** | 1000 ml contain: |
|---|---|
| Sodium chloride | 4.000 g |
| Potassium chloride | 0.050 g |
| Calcium chloride 2 H₂O | 0.066 g |
| Sodium hydrogen carbonate | 0.050 g |
| Taurultam | 1% |

The solution is set at a pH of 7.5 prior to sterilization and subsequently filtered in a sterile manner, deposited in 250 ml infusion bottles and sterilized with steam for 15 minutes at 121°C.

### Use of the Microprobe Apparatus

[0041] The microprobe apparatus can be utilized in, for example, treatment of nonextirpated tumors or reoccurrences as well as in inoperable tumors, e.g., diffuse brain stem gliomas.

In some preferred embodiments, an isotonic solution as described above, can be, e.g., stored at body temperature in a tank. A small pump (which can be located, e.g., subcutaneous or outside the body) can force the Taurolidine and/or Taurultam solution via tubular microprobe to the tumor and/or its surrounding region. In some embodiments, the microprobe may be formed of plastic material with a small lumen. The tip of the probe may have a semipermeable membrane so that an osmotic fluid exchange can occur. In this way, the Taurolidine and/or Taurultam can diffuse inside the tumor and its surroundings. Different types of probes can include a probe with a small tip to terminate directly inside the tumor. With large tumors, a large membrane can be provided at the end of the probe to lie inside the tumor cavity or on the surface of the tumor. In some cases, such as with large tumors, more than one probe or a plurality of probes may be implanted or inserted into the patient.

A prepared solution of taurolidine and/or taurultam is stored in the reservoir. In some embodiments, this is situated out of the body. In some embodiments, it is implanted subcutaneously. Preferably, a small pump (e.g., located external to the body or implanted subcutaneously) forces the solution via one or more microprobes to the tumor and/or infection and/or the surrounding of these processes.

The microprobe(s) include a semipermeable membrane so that an osmotic fluid exchange can occur. Accordingly, the taurolidine and/or taurultam can diffuse into the tumor and/or infection and/or the surrounding of these processes.

In some embodiments, different types of microprobes can be provided. For example, some microprobes can have a small tip that ends directly inside the tumor and/or the infection during use. As another example, other microprobes can include a large membrane at or proximate the end that lies inside the tumor cavity and/or the surface of the tumor and/or infection during use. In some treatment or the like processes (such as, e.g., involving larger regions), a plurality of probes can be implanted. In some processes, the plurality of probes used can include similar and/or dissimilar probes depending on circumstances.

In some preferred embodiments, a probe can be made with high-polymer materials as used in known dialysis devices. In some preferred embodiments, a probe can be made with a plurality of small fibres that together form a bundle to reach a particular size.

In some preferred embodiments, the apparatus can enable treatment or the like processes to be performed In which direct and local application of a drug can be achieved without the need for a carrier.

In some preferred embodiments, the apparatus can enable treatment or the like process to be performed in which blood brain barrier is not or is substantially not a problem to pass.

In some preferred embodiments, the apparatus can be selected, varied and/or adapted based on circumstances in that dependant on the membrane, different molecule sizes are available.

In some preferred embodiments, the apparatus can be substantially self regulating in that, e.g., self regulation of the concentration inside a tumor and/or infection can occur (e.g., inhibiting overdosage).

In some preferred embodiments, the apparatus can achieve a significant reduction in side effects (e.g., to patients).

In some preferred embodiments, the apparatus can achieve a very high local concentration.

In some preferred embodiments, the apparatus can significantly facilitate measurement of concentration.

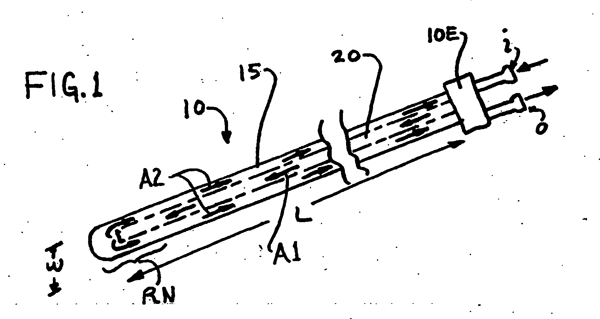

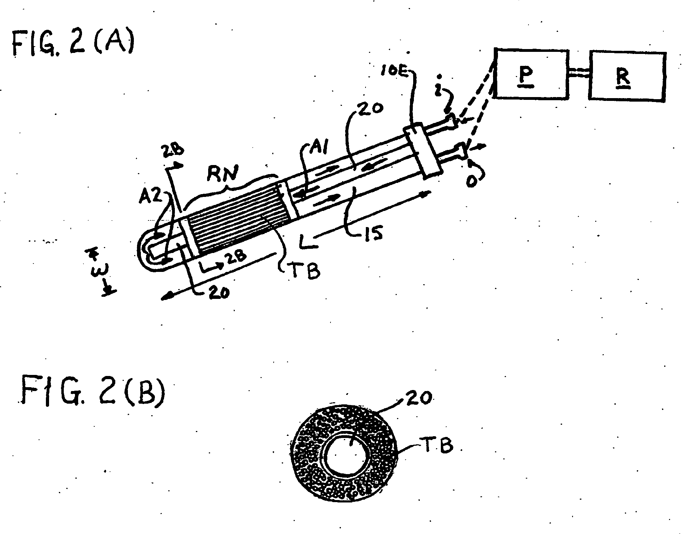

In some embodiments, microprobes of the invention can have a width or diameter (see, e.g., width W shown in FIGS. 1 and 2(A)) of between about 500-1000µm, while in some embodiments, microprobes can have a width or diameter of between about 200-500µm, while in some embodiments, microprobes can have a width or diameter of between about 100-200µm. Other embodiments can have larger or smaller widths or diameters depending on circumstances.

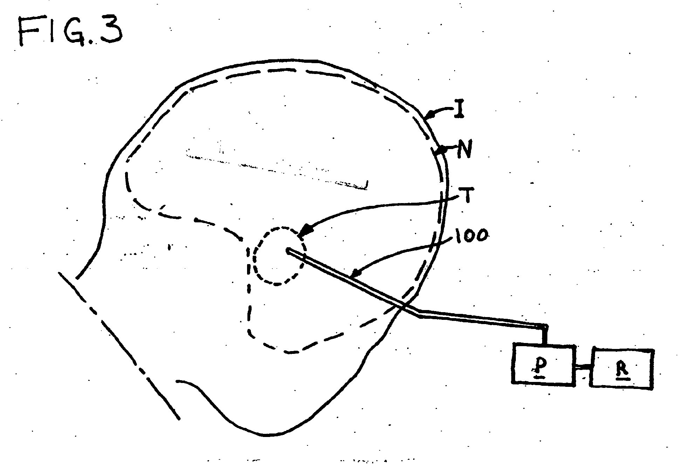

FIG. 1 shows a microdialysis probe 10 according to some illustrative embodiments of the invention. As shown, the probe 10 is preferably an elongated tube-like member with a length L sufficient to extend within a patient (such as, e.g., as shown in FIG. 3) to a desired location and a small width W. Preferably, the probe is a tubular member with a substantially circular cross-section (such as, e.g., as shown in FIG. 3). However, the cross-section can vary in shape depending on circumstances. In preferred embodiments, the probe 10 includes an outer probe delivery region 15 and an internal delivery lumen 20. In some embodiments, the lumen 20 is located substantially along a central axis of the probe and is substantially concentric with the delivery region. However, in other embodiments, the lumen can be situated at other positions or at other locations within or with respect to the probe 10. As shown, by arrows A1, a solution can be delivered via the delivery lumen 20 toward a distal end of the microprobe. Then, as shown by arrows A2 the solution can pass through the delivery region 15 (around the delivery lumen).

As shown, the probe 10 includes a semipermeable region RN through which solution can permeate. In some embodiments, the region RN can be a small region at a tip of the probe 10 (such as, e.g., shown). In some embodiments; the region RN can be set back from the tip of the probe (such as, e.g., shown in FIG. 2). In some embodiments, the region RN can extend a substantial length along the probe. In some illustrative embodiments, the region RN can be less than about 1mm long, in other embodiments it is between about 1-2 mm long, in other embodiments it can be between about 2-4 mm long, in other embodiments, it can be between about 4-8 mm long, in other embodiments it can be longer than 8 mm long. In some preferred embodiments, the region RN is formed by a semipermeable membrane. Various membranes can be selected by those in the art. Notably, dependent on the membrane, different molecule sizes are available. The microprobes can have a variety of different constructions and sizes, which can vary depending on the particular application. In addition, the lengths L and/or RN, the molecular weight cut-off (which is a value less than 5,000 Daltons (g/mol)), and the type of semipermeable membrane used can be selected based on circumstances (such as, e.g., based on characteristics of recovered and/or permeated molecules). Some illustrative semipermeable materials may include one or more of the following materials: cuprophane, polycarbonate, polyethersylfone, polyacrylonitrile; cellulose acetate; regenerated cellulose; and/or various appropriate polymers, hydrophilic membranes, and/or other materials.

In some embodiments, the probes can have a length L of under about 10 cm, while in other embodiments, the length L can be between 10-100 cm, while in other embodiments, the length L can be longer. In some embodiments, the microprobes can be inserted or implanted into a patient using guide cannula or the like. In some embodiments, the microprobes can be substantially rigid, while in other embodiments, the microprobes can be substantially flexible, while in other embodiments, the microprobes can include substantially flexible and/or substantially rigid portions.

In some embodiments, a microdialysis probe provides mass transport in and out of the probe as a function of a concentration gradient across a membrane. Accordingly, the probe can be used to deliver and/or recover compounds from, for example, an extracellular fluid in the local area of implantation surrounding the implanted probe.

FIGS. 2(A) and 2(B) show another illustrative embodiment in which a microprobe includes a semipermeable membrane formed in a region RN. As shown, in this illustrative embodiment, the region RN is set back slightly from a distal tip end of the microprobe. In this illustrative embodiment, the semipermeable membrane is formed by a bundle of small tubes TB.

The bundle of small tubes can include a set of general parallel tubes all penetrating annular binders or plates at either end of the tube bundle TB. The solution material can be pumped through the tubes in a cross-flow manner (such as, e.g., left to right in FIG. 2(A)). Permeate can leave through the sides of the small tubes. On the other hand, retentate can enter the sides of the small tubes and can pass out of the downstream end of the tubes. Among other things, such a design can enhance membrane/solution contact, etc.

As best shown in FIG. 2(A), In the embodiments shown in FIG. 1 and FIGS. 2(A)-2(B), a small pump P can be provided that pumps solution from a reservoir R into the microprobe at an inlet *l.* The inlet *l* can extend to an end member 10E of the microprobe 10 that is configured to direct the solution into the lumen 20. The pump P can also be connected to an outlet *o* such that solution and/or other fluid can be removed from the microprobe. The outlet *o* can similarly extend to the end member 10E and the end member can be similarly configured to connect to the region 15 within the microprobe.

FIG. 3 shows an illustrative use of microprobe apparatus according to the invention in which a prepared isotonic and body-temperature solution is stored in a tank or reservoir R. As shown, a small microdialysis probe or catheter (i.e., microprobe) 100 is implanted inside a patient l (such as, e.g., inserted into the patient's skull and into the patient's central nervous system such as, e.g., within the patients brain N) toward a tumor and/or infection region T. In the embodiment shown in FIG. 3, the microprobe is implanted inside or within the region T. A small pump P can force the solution via at least one microprobe 100.

Microprobes in accordance with the invention can be placed using neuronavigation, MRI guidance and/or ultrasound guidance. A diagnostic biopsy can be taken from the tumor to make a histological diagnosis during the same surgical procedure in which treatment utilizing a microprobe in accordance with the invention is utilized. Alternatively, during use of a microprobe in accordance with the present invention, fluid can be obtained from the tumor or its surroundings so as to maintain a desired fluid level in the area of the tumor.

### Dosage:

The solution for delivery to a patient should contain an effective dosage of Taurolldine and/or Taurultam and/or Taurultam-glucose in the tissue-culture of glioblastoma multiform-tumor cells: as little as 0.1-4 mg/ml Taurolidine inhibits or kills tumor cells in tissue-culture.

Taurultam so far has been shown to be almost twice as effective as Taurolidine, the explanation of which may be found in the equilibrium of Taurolidine in aqueous solution between Methylol-Taurultam and Taurultam.

Taurultam-glucose, on the other hand, has to be dosaged about twice as high as Taurultam, as the molecular weight from Taurultam increases from 136 to 298.

## Claims

1. An apparatus comprising an elongate microprobe (10) for insertion into a patient, the apparatus containing a solution comprising a dissolved agent for delivery to a region (T) of a tumour and/or infection in the central nervous system or other parenchymal organ of said patient, said microprobe (10) having a lumen (20) into which said solution is feedable and a semipermeable region (RN) through which in use said solution may pass to said region (T), said apparatus further comprising a reservoir (R) containing said solution and a pump (P) for pumping said solution from said reservoir (R) into said lumen (20),
(i) said lumen (20) extends lengthwise within the microprobe (10) from inlet means (i) at a base end thereof to an opening proximate the end distal to said base end;
(ii) the microprobe (10) further comprises a return path (15) for said solution and/or other fluid, said return path (15) being positioned around said lumen (20) and extending from said opening to outlet means (o) at said base end;
(iii) the semipermeable region (RN) is positioned around at least a portion of said return path (15);
**characterised in that**:
(iv) the semipermeable region (RN) has a molecular weight cut-off value of less than 5,000 Daltons (g/mol) and
(v) the said dissolved agent is taurolidine, taurultam or a mixture thereof.

2. Apparatus as claimed in claim 1 wherein said semipermeable region (RN) comprises a tube bundle (TB).

3. Apparatus as claimed in claim 1 wherein said semipermeable region (RN) comprises a semipermeable membrane.

4. Apparatus as claimed in any preceding claim wherein said lumen (20) extends substantially concentrically within said microprobe (10).

## Patentansprüche

1. Vorrichtung umfassend eine längliche Mikrosonde (10) zum Einführen in einen Patienten, wobei die Vorrichtung eine Lösung enthält, die ein in Lösung befindliches Mittel zur Zuführung zu einem Bereich (T) eines Tumors und/oder einer Infektion im zentralen Nervensystem oder anderen Epithelzellenorgan des Patienten, wobei die Mikrosonde (10) ein Lumen (20) besitzt, in welches die Lösung einführbar ist, und einen semipermeablen Bereich (RN), durch den hindurch die Lösung während der Benutzung zu dem Bereich (T) gelangen kann, wobei die Vorrichtung desweiteren ein die Lösung enthaltendes Reservoir (R) und eine Pumpe (P) zum Pumpen der Lösung von dem Reservoir (R) in das Lumen (20) umfaßt,
(i) wobei sich das Lumen (20) innerhalb der Mikrosonde (10) in Längsrichtung von einer Einlaßeinrichtung (i) am Basisende davon zu einer Öffnung, die in der Nähe des von dem Basisende entfernten Endes liegt, erstreckt;
(ii) wobei die Mikrosonde (10) desweiteren einen Rückstromweg (15) für die Lösung und/oder anderes Fluid umfaßt, wobei der Rückstromweg (15) um das Lumen (20) herum angeordnet ist und sich von der Öffnung zur Auslaßeinrichtung (o) an dem Basisende erstreckt;
(iii) wobei der semipermeable Bereich (RN) um zumindest einen Teil des Rückstromwegs (15) angeordnet ist;
**dadurch gekennzeichnet, daß**
(iv) der semipermeable Bereich (RN) ein Molekulargewicht-Anhaltewert ("cut-off value") von weniger als 5.000 Daltons (g/mol) besitzt und
(v) das genannte in Lösung befindliche Mittel Taurolidin, Taurultam oder eine Mischung daraus ist.

2. Vorrichtung nach Anspruch 1, wobei der semipermeable Bereich (RN) ein Röhrenbündel (TB) umfaßt.

3. Vorrichtung nach Anspruch 1, wobei der semipermeable Bereich (RN) eine semipermeable Membran umfaßt.

4. Vorrichtung nach einem der voranstehenden Ansprüche, wobei sich das Lumen (20) im wesentlichen konzentrisch innerhalb der Mikrosonde (10) erstreckt.

## Revendications

1. Appareil comprenant une microsonde allongée (10) pour une insertion dans un patient, l'appareil contenant une solution comprenant un agent dissous pour un apport à une région (T) d'une tumeur et/ou d'une infection dans le système nerveux central ou un autre parenchyme dudit patient, ladite microsonde (10) ayant une lumière (20) dans laquelle ladite solution peut être introduite et une région semi-perméable (RN) à travers laquelle ladite solution peut passer à ladite région (T) lors de l'utilisation, ledit appareil comprenant en outre un réservoir (R) contenant ladite solution et une pompe (P) destinée à pomper ladite solution à partir dudit réservoir (R) dans ladite lumière (20),
(i) ladite lumière (20) s'étend en longueur à l'intérieur de la microsonde (10) à partir de moyens d'admission (i) au niveau d'une extrémité de base de celle-ci jusqu'à une ouverture proche de l'extrémité distale de ladite extrémité de base ;
(ii) la microsonde (10) comprend en outre un chemin de retour (15) pour ladite solution et/ou un autre fluide, ledit chemin de retour (15) étant situé autour de ladite lumière (20) et s'étendant à partir de ladite ouverture jusqu'aux moyens d'évacuation (o) au niveau de ladite extrémité de base ;
(iii) la région semi-perméable (RN) est située autour d'au moins une partie dudit chemin de retour (15) ;
**caractérisé en ce que**
(iv) la région semi-perméable (RN) présente une valeur de coupure de poids moléculaire inférieure à 5 000 Daltons (g/mol) et
(v) ledit agent dissous est la taurolidine, le taurultam, ou un mélange de ceux-ci.

2. Appareil selon la revendication 1, dans lequel ladite région semi-perméable (RN) comprend un faisceau tubulaire (TB).

3. Appareil selon la revendication 1, dans lequel ladite région semi-perméable (RN) comprend une membrane semi-perméable.

4. Appareil selon l'une quelconque des revendications précédentes, dans lequel ladite lumière (20) s'étend essentiellement de manière concentrique à l'intérieur de ladite microsonde (10).
